# EUROPÄISCHE PATENTSCHRIFT

(11) **EP 1 170 261 B1**
(45) Veröffentlichungstag und Bekanntmachung des Hinweises auf die Patenterteilung: **05.11.2008**
(21) Anmeldenummer: 01114795.6
(22) Anmeldetag: 27.06.2001
(51) Int. Cl.: C03C 3/078, C03C 3/095, C03C 10/00, C03C 10/16, A61K 6/06

(54) **Tiefsinterndes Kalium-Zink-Silicat-Glas**
Potassium-zinc-silicate glass sintering at low temperature
Verre de potassium-zinc-silicate pouvant fritter à basse température

(30) Priorität: 28.06.2000 DE 10031431
(43) Veröffentlichungstag der Anmeldung: 09.01.2002
(73) Patentinhaber: Ivoclar Vivadent AG, 9494 Schaan (LI)
(72) Erfinder: Schweiger, Marcel, 7000 Chur (CH); Rheinberger, Volker, 9490 Vaduz (LI); Höland, Wolfram, 9494 Schaan (LI)
(74) Vertreter: UEXKÜLL & STOLBERG

(56) Entgegenhaltungen:
- EP-A- 0 885 855
- EP-A- 0 916 625
- US-A- 5 968 856
- US-A- 6 121 175

## Beschreibung

Die Erfindung betrifft Kalium-Zink-Silicat-Glas und insbesondere solches, welches bei niedrigen Temperaturen durch Sinterung verarbeitet werden kann und sich vornehmlich zur gewünschten Einstellung der optischen Eigenschaften und der Verarbeitungseigenschaften von Beschichtungs- und Verblendmaterial für keramische Dentalrestaurationen eignet.

Neben metallischen Dentalrestaurationen, die aus ästhetischen Gründen mit keramischen Schichten verblendet werden, finden in der Zahnheilkunde in zunehmendem Maße vollkeramische Restaurationen Verwendung, bei denen auf einen Kern aus keramischem Werkstoff auch ein keramisches Verblend- oder Beschichtungsmaterial aufgebracht wird. Für den Einsatz als Kern- und auch als Beschichtungsmaterial kommen u.a. Glaskeramiken in Frage.

Vor allem die optischen Eigenschaften sowie die Verarbeitungseigenschaften von glaskeramischem Beschichtungsmaterial sind jedoch vielfach nicht zufriedenstellend. So zeigen die eingesetzten Glaskeramiken infolge ihres hohen Kristallgehaltes eine starke Trübung, die gerade bei Dentalrestaurationen für den Schneidezahnbereich nicht annehmbar ist. Darüber hinaus haben die Glaskeramiken in vielen Fällen einen sehr hohen Ausdehnungskoeffizienten, weshalb sie als Beschichtungsmaterial für Kerne aus Glaskeramik mit niedrigem Ausdehnungskoeffizienten, wie z.B. Lithiumdisilicat-Glaskeramik, ungeeignet sind. Infolge der mangelhaften Anpassung der Ausdehnungskoeffizienten kann es zu unerwünschten Ablösungen des Beschichtungsmaterials kommen.

Weiter ist es bekannt, daß gerade leucithaltige Glaskeramiken sehr hohe lineare thermische Ausdehnungskoeffizienten besitzen. Diese sind auf den Gehalt an Leucit-Kristallen zurückzuführen, die durch gesteuerte Kristallisation eines entsprechenden Ausgangsglases gebildet werden.

Aus der EP-A-695 726 sind Alkali-Zink-Silicat-Gläser bekannt, die zum Verblenden von vornehmlich metallischen Dentalsuprastrukturen geeignet sind, jedoch nur maximal 8,0 Gew.-% ZnO enthalten können, weshalb deren chemische Beständigkeit noch nicht in jedem Falle zufriedenstellend ist. Die Gläser bilden überdies bei Wärmebehandlung im Bereich 600°C bis 1000°C und damit unter für die zahntechnische Weiterverarbeitung üblichen Bedingungen entsprechende Glaskeramiken, die infolge ihres Kristallgehaltes stark getrübt sind und sich daher nicht zur Einstellung einer hohen Transluzenz bei einem glaskeramischen Beschichtungsmaterial eignen. Der Gehalt an Kristallen, insbesondere Leucit, führt außerdem zu unerwünscht hohen Sintertemperaturen und Ausdehnungskoeffizienten, so daß sie für die Verblendung von keramischen Substraten mit niedrigen Ausdehnungskoeffizienten nicht zufriedenstellend sind.

Aus der EP-A-885 606 sind Alkali-Silicat-Gläser bekannt, die als dentale Beschichtungs- oder Verblendmaterialien eingesetzt werden können. Sie haben allerdings lediglich einen Maximalgehalt an ZnO von 5,0 Gew.-% und zeichnen sich u.a. durch einen nur geringen Gehalt an K₂O von maximal 8,5 Gew.-% aus. Als Folge davon ist die chemische Beständigkeit dieser Gläser nicht in jedem Fall ausreichend und sie haben nach wie vor recht hohe Sintertemperaturen.

Auch wenn die bekannten Gläser bereits gute Ergebnisse beim Einsatz als Komponenten von Verblend- und Beschichtungsmaterialien zeigen, so sind jedoch mit ihnen Beschichtungen auf dünnen Gerüsten aus Glaskeramik nicht ohne Rissbildung herstellbar. Bei diesen dünnen Schichtverbunden kommt es in der Regel zu einer Spannungsbildung und damit zu einem Abplatzen des aufgebrachten Beschichtungsmaterials oder zu einem Brechen der fertigen Dentalrestauration. Ein weiterer Grund für dieses Verhalten sind die nach wie vor recht hohen Sintertemperaturen der bekannten Gläser. Daher ist mit ihnen die Herstellung von Veneers, Verblendschalen oder dünnwandigen Kronen mit glaskeramischem Kern nicht möglich.

Weiter ist die zufriedenstellende Verarbeitung der bekannten Gläser durch Sinterung nur in einem engen Temperaturbereich möglich. Bei größeren Abweichungen von der eigentlichen Sintertemperatur zeigen diese Gläser im Falle zu hoher Temperatur eine unbefriedigende Formstabilität und im Falle zu niedriger Temperatur eine inakzeptabel hohe Porosität nach der Sinterung Die zufriedenstellende Verarbeitbarkeit nur in einem engen Temperaturintervall ist sehr nachteilig, da gerade die für die Herstellung von Dentalrestaurationen eingesetzten Öfen klein sind, und es somit generell bei ihnen schwierig ist, eine gewünschte Temperatur über einen gewissen Zeitraum konstant aufrechtzuerhalten. Besonders bei Öfen, die bei niedrigen Temperaturen, wie kleiner als 850°C, arbeiten, treten während eines Sintervorganges erhebliche Temperaturschwankungen auf.

Der Erfindung liegt daher die Aufgabe zugrunde, ein Glas zur Verfügung zu stellen, welches einen niedrigen Ausdehnungskoeffizienten, eine niedrige Sintertemperatur, eine hohe chemische Beständigkeit sowie eine hohe Transluzenz aufweist und dessen chemische Zusammensetzung insbesondere mit der von Apatit- und Lithiumdisilicat-Glaskeramik kompatibel ist, so daß ein fester Verbund zwischen dem Glas und der Glaskeramik gebildet werden kann und das Glas sich somit insgesamt zur Herstellung von Beschichtungen oder Verblendungen bei dünnwandigen dentalen Restaurationen eignet. Weiter soll das Glas in einem breiten Temperaturbereich zu den gewünschten Restaurationen verarbeitbar sein.

Diese Aufgabe wird durch das Kalium-Zink-Silicat-Glas nach den Ansprüchen 1 bis 7 gelöst.

Gegenstand der vorliegenden Erfindung sind ebenfalls das Dentalmaterial nach den Ansprüchen 8 bis 11, die Verwendung nach den Ansprüchen 12 bis 16 sowie das geformte Dentalprodukt nach den Ansprüchen 17 bis 20.

Das erfindungsgemäße Kalium-Zink-Silicat-Glas ist dadurch gekennzeichnet, daß es die folgenden Komponenten enthält:

| Komponente | Gew.-% |
|---|---|
| SiO₂ | 60,0 bis 72,0 |
| Li₂O | 1,0 bis 5,0 |
| K₂O | 10,0 bis 23,0 |
| ZnO | 8,5 bis 20,0. |

Das erfindungsgemäße Glas kann zusätzlich noch mindestens eine der folgenden Komponenten enthalten:

| Komponente | Gew.-% | | |
|---|---|---|---|
| Na₂O | 0 | bis | 4,0 |
| MgO | 0 | bis | 4,0 |
| CaO | 0 | bis | 3,6 |
| SrO | 0 | bis | 3,0 |
| Al₂O₃ | 0 | bis | 8,0 |
| B₂O₃ | 0 | bis | 3,3 |
| La₂O₃ | 0 | bis | 3,0 |
| ZrO₂ | 0 | bis | 6,0 |
| TiO₂ | 0 | bis | 2,5 |
| CeO₂ | 0 | bis | 2,0 |
| SnO₂ | 0 | bis | 5,0 |
| P₂O₅ | 0 | bis | 1,0 |
| Tb₄O₇ | 0 | bis | 1,8 |
| F | 0 | bis | 1,1. |

Sofern diese zusätzlichen Komponenten vorhanden sind, werden sie insbesondere in Mengen von mindestens 0,1 Gew.-% eingesetzt. Für die einzelnen Komponenten des erfindungsgemäßen Kalium-Zink-Silicat-Glases existieren bevorzugte Mengenbereiche. Diese können unabhängig voneinander gewählt werden, und sind wie folgt:

| Komponente | Gew.-% | | |
|---|---|---|---|
| SiO₂ | 62,0 | bis | 70,0 |
| Li₂O | 2,0 | bis | 5,0 |
| K₂O | 10,0 | bis | 20,0 |
| ZnO | 10,0 | bis | 19,0 |
| Na₂O | 0 | bis | 3,0 |
| MgO | 0 | bis | 3,0 |
| CaO | 0 | bis | 3,0 |
| SrO | 0 | bis | 3,0 |
| Al₂O₃ | 0 | bis | 6,0 |
| B₂O₃ | 0 | bis | 3,0 |
| La₂O₃ | 0 | bis | 2,0 |
| ZrO₂ | 0 | bis | 5,0 |
| TiO₂ | 0 | bis | 2,0 |
| CeO₂ | 0 | bis | 1,5 |
| SnO₂ | 0 | bis | 4,0 |
| P₂O₅ | 0 | bis | 0,8 |
| Tb₄O₇ | 0 | bis | 1,0 |
| F | 0 | bis | 1,0 |

Besonders bevorzugte Mengenbereiche für die einzelnen Komponenten des erfindungsgemäßen Glases sind wie folgt, und diese können ebenfalls unabhängig voneinander gewählt werden:

| Komponente | Gew.-% | | |
|---|---|---|---|
| SiO₂ | 63,0 | bis | 69,0 |
| Li₂O | 3,0 | bis | 5,0 |
| K₂O | 11,0 | bis | 19,0 |
| ZnO | 10,0 | bis | 17,0 |
| Na₂O | 0 | bis | 2,5 |
| MgO | 0 | bis | 2,5 |
| CaO | 0 | bis | 2,5 |
| SrO | 0 | bis | 2,5 |
| Al₂O₃ | 0 | bis | 4,0 |
| B₂O₃ | 0 | bis | 2,0 |
| La₂O₃ | 0 | bis | 1,8 |
| ZrO₂ | 0 | bis | 4,0 |
| TiO₂ | 0 | bis | 1,8 |
| CeO₂ | 0,1 | bis | 1,5 |
| SnO₂ | 0 | bis | 3,5 |
| P₂O₅ | 0 | bis | 0,5 |
| Tb₄O₇ | 0 | bis | 0,8 |
| F | 0 | bis | 0,8 |

Alle vorstehenden Mengenangaben in Gew.-% beziehen sich auf das Glas.

Zur Herstellung des erfindungsgemäßen Glases wird vorzugsweise so vorgegangen, daß geeignete Ausgangsmaterialien, wie z.B. Carbonate, Oxide und Fluoride, bei einer Temperatur im Bereich von 1350 °C bis 1650 °C, vorzugsweise 1400 °C bis 1600 °C, über einen Zeitraum von 30 Minuten bis 4 Stunden, vorzugsweise eine Stunde bis 2,5 Stunden, unter Bildung einer homogenen Schmelze erschmolzen werden. Das erschmolzene Glas wird dann üblicherweise in Wasser abgeschreckt, d.h. gefrittet, und nach Trocknen auf die gewünschte Korngröße gemahlen.

Mittels rasterelektronenmikroskopischer Untersuchungen wurde festgestellt, daß das erfindungsgemäße Glas frei von Kristallen ist. Weiter zeigte sich, daß das Glas auch die bei einer üblichen zahntechnischen Weiterverarbeitung durch Sinterung herrschenden Bedingungen übersteht, ohne daß es zu der bei bekannten Gläser auftretenden Bildung von Kristallen kommt. Selbst bei einer thermischen Behandlung im Bereich von 600 °C bis 800 °C für 1 Minute bis 1 Stunde trat eine Kristallisation nicht auf.

Das erfindungsgemäße Glas hat üblicherweise beim Aufsintern auf ein keramisches oder glaskeramisches Substrat, wie eine Lithiumdisilikat-Glaskeramik eine sehr vorteilhafte Sintertemperatur von weniger als 800°C. Besonders bevorzugt sind solche Gläser, die ein Sintertemperatur von 760°C und weniger haben und damit bei dieser Temperatur verarbeitet werden können. Diese niedrigen Sintertemperaturen sind vermutlich auf die spezielle Zusammensetzung des erfindungsgemäßen Glases zurückzuführen.

Es ist von ganz besonderem Vorteil, dass das erfindungsgemäße Glas auch noch bei großen Abweichungen von der eigentlichen Sintertemperatur, d.h. der Temperatur, bei der die Formstabilität sowie die Porosität des Glases besonders zufriedenstellend sind, durch Sinterung verarbeitet werden kann. So kann das Glas sogar in einem Sintertemperaturbereich von ± 20°C, oder mehr, wie z.B. ± 40°C, ober- bzw. unterhalb der eigentlichen Sintertemperatur verarbeitet werden, ohne dass Risse oder Sprünge in der Dentalrestauration auftreten. Bei einer Verarbeitung in diesem Temperaturbereich weist das gesinterte Glas eine sehr geringe Porosität und eine sehr gute Formstabilität auf. Die ausgezeichnete Formstabilität zeigt sich daran, dass selbst die sehr dünnwandige Zahnschneidekante, die durch Aufbringen eines Gemisches aus Glaspulver und Anmischflüssigkeit auf ein Gerüst sowie dessen Formung gebildet wurde, nach dem Sintervorgang ihre Form behält und damit bestehen bleibt. Damit kann das erfindungsgemäße Glas auch in solchen Öfen gesintert werden, die eine genaue Konstanthaltung der Brenntemperatur nicht gestatten, was ein besonderer Vorteil ist. Demgegenüber gestatten konventionelle Gläser lediglich Abweichungen von ± 10°C von der Sintertemperatur. Bei größeren Abweichungen sind mit ihnen zufriedenstellende Restaurationen nicht herstellbar.

Zur Durchführung der Sinterung des erfindungsgemäßen Glases wird in der Regel eine Aufheizrate von 3°C bis 100°C/min und bevorzugt von 30°C bis 80°C/min sowie eine Haltezeit bei der Sintertemperatur von 10 Sekunden bis 1 Stunde und bevorzugt 30 Sekunden bis 5 Minuten gewählt. Dabei ist es vorteilhaft, die Sinterung im Vakuum durchzuführen, damit der Sinterkörper möglichst wenig Poren aufweist.

Der lineare thermische Ausdehnungskoeffizient des erfindungsgemäßen Glases beträgt üblicherweise weniger als 12,3 × 10⁻⁶K⁻¹, bevorzugt 7,7 bis 10,9 × 10⁻⁶K⁻¹, gemessen im Temperaturintervall von 100 °C bis 400 °C.

Das erfindungsgemäße Glas wird entweder alleine oder zusammen mit weiteren Komponenten bevorzugt als Dentalmaterial eingesetzt. Hierzu wird es üblicherweise in Form eines Pulvers mit einer mittleren Teilchengröße von weniger als 90 µm verwendet. Als weitere Komponenten kommen Glaskeramiken und andere Gläser, aber auch Farbstoffe, insbesondere Farbpigmente, Oxide der 3d-Elemente oder Metallkolloide, sowie Fluoreszenzstoffe, insbesondere mit d- und f-Elementen dotiertes Ytterbium-Silicat, in Frage.

Besonders vorteilhaft ist Dentalmaterial, das als weitere Komponente mindestens eine Glaskeramik und bevorzugt eine Apatit-Glaskeramik enthält.

Dabei ist eine Apatit-Glaskeramik bevorzugt, die die folgenden Komponenten enthält und bei der die Hauptkristallphase durch Apatit-Kristalle gebildet ist:

| Komponente | Gew.-% | | |
|---|---|---|---|
| SiO₂ | 56,0 | bis | 65,0 |
| Li₂O | 1,8 | bis | 5,3 |
| K₂O | 9,0 | bis | 17,5 |
| ZnO | 9,0 | bis | 16,0 |
| CaO | 3,5 | bis | 10,5 |
| P₂O₅ | 2,0 | bis | 6,0 |
| F | 0,5 | bis | 1,0 |

Besonders bevorzugt enthält diese Apatit-Glaskeramik zusätzlich mindestens eine der folgenden Komponenten:

| Komponente | Gew.-% | | |
|---|---|---|---|
| Na₂O | 0 | bis | 5,0 |
| MgO | 0 | bis | 3,5 |
| SrO | 0 | bis | 3,5 |
| Al₂O₃ | 0 | bis | 6,0 |
| B₂O₃ | 0 | bis | 2,0 |
| La₂O₃ | 0 | bis | 3,0 |
| ZrO₂ | 0 | bis | 7,5 |
| TiO₂ | 0 | bis | 7,5 |
| CeO₂ | 0 | bis | 2,0 |
| SnO₂ | 0 | bis | 5,0 |
| Tb₄O₇ | 0 | bis | 0,5 |

Vorstehenden Angaben in Gew.-% beziehen sich auf die Apatit-Glaskeramik. Sofern diese zusätzlichen Komponenten vorhanden sind, werden sie insbesondere in Mengen von mindestens 0,1 Gew.-% eingesetzt.

Die Apatit-Glaskeramiken werden hergestellt, indem aus geeigneten Ausgangsmaterialien, wie Oxiden, Carbonaten und Fluoriden, ein Ausgangsglas bei Temperaturen von 1200°C bis 1650 °C erschmolzen wird, dieses in Wasser eingegossen wird und das gebildete Glasgranulat, ggf. nach weiterem Zerkleinern, einer thermischen Behandlung von mehr als 500°C und bis zu 900°C für eine Dauer von 30 Minuten bis 6 Stunden unterzogen wird.

Die erhaltenen Apatit-Glaskeramiken zeichnen sich durch eine hohe Transluzenz, hohe chemische Beständigkeit sowie einen niedrigen Ausdehnungskoeffizienten aus. Sie sind darüberhinaus in ihrer chemischen Zusammensetzung ausgezeichnet an die erfindungsgemäßen Gläser angepaßt, so daß nachteilige Stofftransportreaktionen zwischen beiden Materialien und damit einhergehende Spannungsbildung gerade bei dünnen Schichtverbunden vermieden werden.

Das erfindungsgemäße Dentalmaterial hat normaleweise einen linearen thermischen Ausdehnungskoeffizienten von 9,0 bis 10,9 x 10⁻⁶ K⁻¹, gemessen im Bereich von 100 °C bis 400 °C. Der jeweils gewünschte Koeffizient kann dabei durch geeignete Wahl der Art des Kalium-Zink-Silicat-Glases und etwaiger weiterer Komponenten, sowie deren Mengen eingestellt werden. Günstige Dentalmaterialen enthalten 10 bis 90 Gew.-% Kalium-Zink-Silicat-Glas und 90 bis 10 Gew.-% weitere Komponenten, bezogen auf das Dentalmaterial.

Das erfindungsgemäße Dentalmaterial eignet sich zur Beschichtung von Substraten und insbesondere zur Beschichtung oder Verblendung von dentalen Restaurationen. Die Beschichtung erfolgt dabei insbesondere durch Aufbringen des Dentalmaterials auf das ausgewählte Substrat und anschließendes Sintern bei weniger als 800°C und insbesondere 760°C oder weniger.

Bevorzugt wird dabei zunächst ein Pulver des erfindungsgemäßen Glases mit einem Pulver der ggf. vorhandenen weitere Komponenten gemischt und durch Zugabe von wäßrigen Anmischlösungen zu einer Paste verarbeitet. Diese Paste wird dann auf das Substrat aufgebracht, und nach gewünschter Formgebung erfolgt das Sintern, um eine fest haftende Beschichtung oder Verblendung zu erhalten.

Das erfindungsgemäße Dentalmaterial kann als Beschichtungs- oder Verblendmaterial für Substrate, wie Dentalsuprastrukturen, z.B. auf Basis von keramischen oder glaskeramischen Werkstoffen eingesetzt werden. Aufgrund seines niedrigen Ausdehnungskoeffizienten wird es bevorzugt bei Substratwerkstoffen mit einem thermischen Ausdehungskoeffizienten von 7,0 bis 12,0, insbesondere von 8,0 bis 11,0 × 10⁻⁶K⁻¹, verwendet. Bevorzugt wird es zur Beschichtung oder Verblendung von ZrO₂-Keramiken, Al₂O₃-Keramiken, ZrO₂/Al₂O₃-Keramiken, keramischen oder glaskeramischen Compositwerkstoffen und Titan verwendet.

Besonders vorteilhaft wird es jedoch zur Verblendung von Substraten auf Basis von Lithiumdisilicat-Glaskeramik eingesetzt, um auf diese Weise ästhetisch sehr ansprechende vollkeramische Dentalprodukte herzustellen, die eine sehr hohe Festigkeit sowie eine ausgezeichnete chemische Beständigkeit haben.

Als besonders geeignet haben sich dabei Lithiumdisilicat-Glaskeramiken erwiesen, die die nachstehend angegebenen Komponenten enthalten und die z.B. durch Erschmelzen entsprechender Ausgangsgläser, Fritten und Wärmebehandlung bei 400 °C bis 1100 °C erhalten werden können:

| Komponente | Gew.-% | | |
|---|---|---|---|
| SiO₂ | 57,0 | bis | 80,0 |
| Al₂O₃ | 0 | bis | 5,0 |
| La₂O₃ | 0,1 | bis | 6,0 |
| MgO | 0 | bis | 5,0 |
| ZnO | 0 | bis | 8,0 |
| Li₂O | 11,0 | bis | 19,0 |
| P₂O₅ | 0 | bis | 11,0 |

wobei
(a) Al₂O₃ + La₂O₃ 0,1 bis 7,0 Gew.-% und
(b) MgO + ZnO 0,1 bis 9,0 Gew.-%
   ausmachen.

Die Angaben in Gew.-% beziehen sich auf die Lithiumdisilicat-Glaskeramik.

Das erfindungsgemäße Kalium-Zink-Silicat-Glas und das erfindungsgemäße Dentalmaterial können zusammen mit den ggf. vorhandenen Zusätzen zu geformten Dentalprodukten in üblicher Weise verarbeitet werden. Als erfindungsgemäße geformte Dentalprodukte, die einen Gehalt an dem Kalium-Zink-Silicat-Glas oder dem Dentalmaterial aufweisen, kommen insbesondere Dentalrestaurationen, wie z.B. ein Inlay, ein Onlay, eine Brücke, ein Stiftaufbau, eine Verblendung, eine Facette, eine Füllung oder ein Verbinder in Frage. Besonders bevorzugte Dentalrestaurationen sind Schalen, Veneers, Brücken, Kronen und Teilkronen.

Die Dentalprodukte haben bevorzugt einen Kern auf Basis von keramischem oder glaskeramischem Werkstoff, insbesondere Lithiumdisilicat-Glaskeramik, auf den das erfindungsgemäße Glas oder das erfindungsgemäße Dentalmaterial aufgebracht ist. Bevorzugte Lithiumdisilicat-Glaskeramiken sind oben bereits beschrieben worden.

Im Gegensatz zu konventionellem Glas ist das erfindungsgemäße Glas in seiner chemischen Zusammensetzung noch besser an Apatit-Glaskeramiken und an Lithiumdisilicat-Glaskeramiken angepaßt, die bevorzugt als weitere Komponente eines Beschichtungsmaterials oder als Substrat eingesetzt werden. Das hat zur Folge, daß gerade bei dünnen Schichtverbunden, wie. z.B. Verblendschalen, mit Lithiumdisilicat-Glaskeramik als Substrat, auf das eine Mischung aus erfindungsgemäßem Glas und Apatit-Glaskeramik aufgebracht wurde, es nicht zu Ablösungserscheinungen der Beschichtung oder einem Brechen des fertigen Produktes kommt. Für dieses vorteilhafte Verhalten ist auch die niedrige Sintertemperatur des erfindungsgemäßen Glases verantwortlich.

Weiter führen die bei der Sinterung des Glases herrschenden Bedingungen nicht zu einer Kristallisation, die seine Transluzenz unerwünscht erniedrigen würde. Damit gibt es im wesentlichen die Färbung des beschichteten Substrates wieder, was gerade bei der Herstellung von vollkeramischen Dentalrestaurationen von besonderem Vorteil ist.

Überdies zeigt das erfindungsgemäße Glas eine ausgezeichnete chemische Beständigkeit, die für seine Verwendung als Dentalmaterial unerläßlich ist, welches in der Mundhöhle permanent von sauren Flüssigkeiten umspült wird. Es ist überraschend, daß das Glas sowohl eine gute chemische Beständigkeit als auch eine niedrige Sintertemperatur aufweist. Möglicherweise ist diese günstige Kombination von Eigenschaften darauf zurückzuführen, daß das Glas gleichzeitig mehrere Arten von Alkalimetall-Ionen enthält.

Die Erfindung wird nachstehend anhand von Beispielen näher erläutert.

### Beispiele

### Beispiele 1 bis 34

Es wurden insgesamt 34 verschiedene erfindungsgemäße Gläser mit den in Tabelle I angegebenen chemischen Zusammensetzungen hergestellt.

**Tabelle I: Zusammensetzung erfindungsgemäßer Kalium-Zink Silicatgläser (Angaben in Gew.-%)**

| *Beispiel* | *SiO*₂ | *Li*₂*O* | *K*₂*O* | *ZnO* | *Na*₂*O* | *MgO* | *CaO* | *SrO* | *B*₂*O*₃ | *Al*₂*O*₃ | *La*₂*O*₃ | *TiO*₂ | *ZrO*₂ | *SnO*₂ | *CeO*₂ | *P*₂*O*₅ | *Tb*₄*O*₇ | *F* |
|---|---|---|---|---|---|---|---|---|---|---|---|---|---|---|---|---|---|---|
| *1* | *63,8* | *3,7* | *18*,*8* | *12,7* | | | | | | | | | | | | *1,0* | | |
| *2* | *67,1* | *4,8* | *15,1* | *13,0* | | | | | | | | | | | | | | |
| *3* | *64,8* | *4,6* | *14,5* | *12,5* | | | *3,6* | | | | | | | | | | | |
| *4* | *65,0* | *4,7* | *14,7* | *12,6* | | | | | | | *3,0* | | | | | | | |
| *5* | *68,9* | *4,3* | *13,5* | *13,3* | | | | | | | | | | | | | | |
| *6* | *68,1* | *3,8* | *14,9* | *13,2* | | | | | | | | | | | | | | |
| *7* | *69,5* | *4,8* | *12,2* | *13,5* | | | | | | | | | | | | | | |
| *8* | *69,5* | *4,8* | *15,2* | *10,5* | | | | | | | | | | | | | | |
| *9* | *67,1* | *4,3* | *13,4* | *13,2* | | | | | | | | | *2,0* | | | | | |
| *10* | *65,4* | *3,7* | *14,7* | *13,0* | | | | | | *3,2* | | | | | | | | |
| *11* | *69,0* | *5,0* | *15,4* | *8,0* | | *2,6* | | | | | | | | | | | | |
| *12* | *68,1* | *4,8* | *15,1* | *8,0* | | *4,0* | | | | | | | | | | | | |
| *13* | *64,8* | *4,7* | *14,7* | *12,7* | | | | | | | | | | | *1,3* | | *1,8* | |
| *14* | *67,2* | *3,7* | *11,8* | *13,0* | | | | | | | | | *1,9* | *2,4* | | | | |
| *15* | *66,7* | *3,7* | *11,7* | *12,9* | | | | | | | | | | *5,0* | | | | |
| *16* | *65,3* | *4,7* | *14,7* | *12,7* | | | | | | | | | | | *2,0* | | *0,6* | |
| *17* | *62,0* | *1,0* | *17,0* | *20,0* | | | | | | | | | | | | | | |
| *18* | *70,6* | *3,8* | *12,0* | *13,6* | | | | | | | | | | | | | | |
| *19* | *67*,*5* | *3,6* | *11,3* | *13,6* | *4,0* | | | | | | | | | | | | | |
| *20* | *72,0* | *3,3* | *10,0* | *14,0* | *0,7* | | | | | | | | | | | | | |
| *21* | *64,0* | *4,0* | *12,6* | *12,4* | | | | | | *3,2* | | | *3,8* | | | | | |
| *22* | *67,1* | *4,2* | *13,2* | *13,0* | | | | | | | | *2,5* | | | | | | |
| *23* | *61,6* | *3,1* | *15,0* | *12,3* | | | | | | *8,0* | | | | | | | | |
| *24* | *60,8* | *3,7* | *23,0* | *12,5* | | | | | | | | | | | | | | |
| *25* | *60,0* | *3,7* | *23,0* | *12,5* | | *0,8* | | | | | | | | | | | | |
| *26* | *68,4* | *3,8* | *12,0* | *13,6* | | | | | *2,2* | | | | | | | | | |
| *27* | *67,8* | *3,8* | *11,9* | *13,5* | | | | *3,0* | | | | | | | | | | |
| *28* | *67,0* | *3,9* | *12,4* | *13,3* | | | *2,3* | | | | | | | | | | | *1,1* |
| *29* | *64,8* | *4,0* | *12,7* | *12,5* | | *0,6* | | *3,0* | | | | | *1,9* | | *0,5* | | | |
| *30* | *65,2* | *4,2* | *14,8* | *12,8* | | *0,6* | | | | | | | *1,9* | | *0,5* | | | |
| *31* | *61,8* | *4,1* | *14,5* | *12,5* | | *0,6* | | | | | | | *6,0* | | *0,5* | | | |
| *32* | *65,3* | *4,5* | *14,2* | *12,9* | | 0,6 | | | | | | | *2,0* | | *0,5* | | | |
| *33* | *64,5* | *4,5* | *14,2* | *12,9* | | | | | *3,3* | | | | | | *0,6* | | | |
| *34* | *65,5* | *4,6* | *14,5* | *11,3* | | *0,6* | | | *1,1* | | | | *1,9* | | *0,5* | | | |

Zu ihrer Herstellung wurde jeweils ein entsprechendes Gemenge von geeigneten Oxiden, Carbonaten und Fluoriden in einem Platin/Rhodium-Tiegel bei einer Temperatur von 1550°C bis 1600°C während einer Homogenisierungszeit von 1 bis 1,5 Stunden erschmolzen. Die Glasschmelze wurde in Wasser abgeschreckt, und das gebildete Granulat des Glases wurde getrocknet und auf eine mittlere Korngröße von weniger als 90 µm, bezogen auf die Anzahl der Teilchen, aufgemahlen.

Für einige der hergestellten Gläser sind in Tabelle II ausgewählte Eigenschaften angegeben, die an Probekörpern aus dem jeweiligen Glas bestimmt worden sind. Die Beispiele verdeutlichen wie durch Veränderung der chemischen Zusammensetzung Gläser mit unterschiedlichen Eigenschaften erhalten werden können.

**Tabelle II: Eigenschaften erfindungsgemäßer Kalium-Zink-Silicatgläser**

| **Beispiel Nr.** | **α-Wert x 10⁻⁶K⁻¹ [100-400°C]** | **Tg[°C]** | **Optik** | **Brenntemp. auf Krone [°C]** | **Säurebeständigkeit [µg/cm²]** | **Brenntemp. für Prütkörper- herstellung [°C]** |
|---|---|---|---|---|---|---|
| 2 | 9,58 | 478 | transparent | 700 | 55 | 780 |
| 7 | 8,76 | 488 | transparent | 720 | 9 | 790 |
| 9 | 8,94 | 500 | transparent | 720 | 13 | 800 |
| 20 | 7,79 | 518 | transparent | 760 | 5 | 840 |
| 27 | 8,64 | 497 | transparent | 720 | 4 | 800 |
| 30 | 9,66 | 504 | transparent | 740 | 13 | 820 |
| 34 | 9,69 | | transparent | 700 | 36 | 780 |
| 1 | 10,57 | 484 | transparent | 710 | 47 | 790 |

### Bestimmung des Ausdehnungskoeffizienten α

Zur Messung des linearen thermischen Ausdehnungskoeffizienten α wurde aus dem Pulver des jeweiligen Glases ein stäbchenförmiger Grünkörper hergestellt, der in einem Vakuumbrennofen mit einer Aufheizrate von 60°C/min und einer Haltezeit von 1 Minute bei der jeweiligen Brenntemperatur für die Prüfkörperherstellung gesintert wurde. Anschließend wurde ein Glanzbrand ohne Vakuum bei einer um 20°C höheren Endtemperatur und einer Haltezeit von 1 Minute durchgeführt. An dem erhaltenen Probekörper wurde der lineare thermische Ausdehnungskoeffizient im Temperaturbereich von 100 bis 400°C bestimmt.

### Bestimmung der Säurebeständigkeit

Die Säurebeständigkeit ist ein Maß für die chemische Beständigkeit gerade von im Dentalbereich eingesetzten Gläsern und Glaskeramiken, da diese in der Mundhöhle permanent der Einwirkung von sauren Substanzen ausgesetzt sind.

Die Säurebeständigkeit wurde gemäß der ISO-Vorschrift 6872:1995 bestimmt. Dazu wurden zunächst Probeplättchen mit einem Durchmesser von 12 mm und einer Dicke von 1 mm durch Zusammensintern von Glas-Pulver mit einer mittleren Teilchengröße von weniger als 90 µm hergestellt. Das Pulver wurde 1 Minute lang auf der jeweiligen Brenntemperatur für die Prüfkörperherstellung gehalten. Dann wurden die Probeplättchen 16 Stunden lang mit 4 Vol.-%iger wäßriger Essigsäure bei 80°C behandelt, und es wurde schließlich der eingetretene Masseverlust bezogen auf die Plättchenoberfläche als Mass für die Säurebeständigkeit bestimmt.

### Beispiel 35

Diese Beispiel beschreibt die Herstellung eines erfindungsgemäßen Glases, das als tiefschmelzendes Glasur- oder Korrekturmaterial verwendet werden kann.

Von besonderem Vorteil ist die Aufbringung einer Glasurschicht mit Gehalt an erfindungsgemäßem Glas auf ein transluzentes Lithiumdisilicat-Grundgerüst, da damit ästhetische Dentalrestaurationen, z.B. in Form von Inlays, Onlays, Teilkronen, Veneers, Kronen oder Brücken herstellbar sind. Durch den Einsatz einer Glasurschicht ist die Aufbringung einer dicken, mehrlagigen Schichtglaskeramik nicht erforderlich.

Zunächst wurde Glaspulver mit der in Tabelle I für Beispiel 34 angegebenen Zusammensetzung analog der oben für die Beispiele 1 bis 34 angegebenen Weise hergestellt. Dieses Pulver wurde zu einem stäbchenförmigen Grünkörper in einem Vakuumofen bei einer Aufheizgeschwindigkeit von 60°C/min und einer Haltezeit von 1 min bei 780°C gesintert. Anschließend wude ein Glanzbrand ohne Vakuum bei 760°C und einer Haltezeit von 1 Minute durchgeführt.

Für die so erhaltene Probe wurde ein thermischer Ausdehnungskoeffizient von 9,69 x 10⁻⁶K⁻¹, gemessen im Temperaturbereich von 100 bis 400°C, bestimmt.

Dieses Glas konnte daher zum Aufsintern auf ein Substrat mit einem niedrigen Ausdehnungskoeffizienten von 10,6 x 10⁻⁶K⁻¹, wie einem Kronen- oder Brückengerüst auf Basis von Lithiumdisilicat-Glaskeramik, verwendet werden. Dabei zeigt sich, daß das Aufsintern des Glases auf das Gerüst bereits bei einer Temperatur von lediglich 700°C möglich war.

Durch den an Lithiumdisilicat-Glaskeramiken angepassten Ausdehnungskoeffizienten, die sehr gute chemische Beständigkeit und die niedrige Verarbeitungstemperatur ist dieses erfindungsgemäße Glas besonders zum Glasieren von sehr transluzenten Gerüsten auf Basis von Lithiumdisilicat-Glaskeramik sowie als Korrekturmaterial für auf solche Gerüste aufgeschichtete Sintermaterialien geeignet.

### Beispiel 36

Dieses Beispiel beschreibt den Einsatz einer Mischung des erfindungsgemäßen Glases nach Beispiel 30 zusammen mit einer Apatit-Glaskeramik als Beschichtungsmaterial für keramische Suprastrukturen und somit dessen Einsetzbarkeit bei der Herstellung von vollkeramischen Dentalprodukten.

Die verwendete Apatit-Glaskeramik hatte die Zusammensetzung (in Gew.-%):
SiO₂ 59,6; Li₂O 4,2; K₂O 13,4; ZnO 10,4;
P₂O₅ 3,5; CaO 6,0; F 0,5; ZrO₂ 1,9; CeO₂ 0,5

Zu Herstellung dieser Apatit-Glaskeramik wurde ein Ausgangsglas entsprechender Zusammensetzung erschmolzen, gefrittet und zu einem Pulver aufgemahlen. Dieses Pulver wurde dann 4 h lang bei 520°C und anschließend 1 h lang bei 800°C wärmebehandelt.

Zum Erhalt eines Beschichtungsmaterials, bei dem Sintertemperatur und Ausdehnungskoeffizienten geeignet eingestellt sind, wurden 50 Gew.-% der Apatitglaskeramik mit 50 Gew.-% des erfindungsgemäßen Glases in Form von Pulvern mit einer mittleren Teilchengröße von weniger als 90µm gemischt.

Diese Mischung wurde zu einem stäbchenförmigen Grünkörper in einem Vakuumofen bei einer Aufheizgeschwindigkeit von 60°C/min und einer Haltezeit von 1 min bei 840°C gesintert. Anschließend wurde ein Glanzbrand ohne Vakuum bei 820°C und einer Haltezeit von 1 Minute durchgeführt. Für die so erhaltene Probe wurde ein thermischer Ausdehnungskoeffizient von 9,96 x 10⁻⁶K⁻¹, gemessen im Temperaturbereich von 100°C bis 400°C, bestimmt.

Damit konnte diese Mischung zum Aufsintern auf eine sehr transluzente Lithiumdisilicat-Glaskeramik mit einem thermischen Ausdehnungskoeffizienten von 10,6 x 10⁻⁶K⁻¹ verwendet werden. Dabei zeigte sich, daß das Aufsintern der Mischung bereits bei einer Temperatur von lediglich 730°C möglich war. Insgesamt konnten somit vollkeramische Dentalprodukte, wie Kronen oder Brücken, hergestellt werden, die sich durch einen ausgezeichneten Verbund der einzelnen Schichten, ein ästhetisches Aussehen und gute chemische Beständigkeit auszeichnen.

### Beispiel 37

### Herstellung einer Verblendschale

Es wurde aus einer Lithiumdisilicat-Glaskeramik durch Verpressen im viskosen Zustand eine Verblendschale für einen mittleren oberen Schneidezahn mit einer Schichtdicke von max. 0,5 mm hergestellt. Nach dem Heißpressen wurde die Schichtstärke durch mechanische Nachbearbeitung mit einem Diamantwerkzeug auf max. 0,25 mm reduziert. Danach wurde die Verblendschale in einer wässrigen Lösung von 0,5 Vol.-% HF und 3 Vol.-% H₂SO₄ während 10 Minuten im Ultraschallbad oberflächlich gereinigt und dann mit Al₂O₃ bei einem Strahldruck von 1,5 bar sandgestrahlt. Danach wurde ein Dentalmaterial aufgesintert, und zwar eine Mischung aus dem erfindungsgemäßen Glas 21 und einer Apatit-Glaskeramik. Die verwendete Apatit-Glaskeramik hatte die Zusammensetzung (in Gew.-%) :
SiO₂: 61,4; Li₂O: 4,3; K₂O: 13,6; ZnO: 10,6; P₂O₅: 3,5; CaO: 6,1; F: 0,5.

Zur Herstellung dieser Glaskeramik wurde ein Ausgangsglas entsprechender Zusammensetzung erschmolzen, gefrittet und zu einem Pulver aufgemahlen. Dieses Pulver wurde dann 1 Stunde lang bei 800°C wärmebehandelt. Zum Erhalt eines Beschichtungsmaterials, bei dem Sintertemperatur und Ausdehnungskoeffizient geeignet eingestellt sind, wurden 50 Gew.-% der Apatit-Glaskeramik mit 50 Gew.-% des erfindungsgemäßen Glases in Form von Pulvern mit einer mittleren Teilchengröße von weniger als 90 µm gemischt. Der thermische Ausdehnungskoeffizient dieses Dentalmaterials betrug 9,4 × 10⁻¹⁰K⁻¹. Die Sintertemperatur lag bei 750°C und wurde bei der Beschichtung der Verblendschale während 1 Minute gehalten. Insgesamt wurden 5 Brände mit Materialauftrag bei 750°C bis zur Fertigstellung der Verblendschale durchgeführt. Der abschließende Glanzbrand wurde bei 740°C ohne Vakuum durchgeführt, um einen oberflächlichen Eigenglanz zu erzielen. Die eine Dentalrestauration darstellende Verblendschale zeigte einen sehr homogenen Schichtverbund. Es bildeten sich keine Risse oder Sprünge, was bei so dünnen Wandstärken nicht selbstverständlich ist. Die Verblendschale zeigte weiter eine sehr gute Transluzenz, was bei dieser Form von Dentalrestauration eine überaus wichtige Eigenschaft ist.

### Beispiel 38

### Herstellung einer 3-gliedrigen Frontzahnbrücke

Es wurde aus einer Lithiumdisilikat-Glaskeramik durch Verpressen im viskosen Zustand ein Frontzahnbrückengerüst mit einem Zwischenglied hergestellt. Die kleinste Wandstärke betrug ca. 0,5 mm. Nach dem Heißpressen wurde das Gerüst mit einer wässrigen Lösung von 0,5 Vol.-% HF und 3 Vol.-% H₂SO₄ während 10 Minuten im Ultraschallbad oberflächlich gereinigt und anschließend mit Al₂O₃ bei einem Strahldruck von 1,5 bar sandgestrahlt. Danach wurde ein Dentalmaterial aufgesintert, welches aus dem erfindungsgemäßen Glas 30 und einer Apatit-Glaskeramik bestand. Die verwendete Apatit-Glaskeramik hatte die Zusammensetzung (in Gew.-%):
SiO₂: 57,8; Li₂O: 4,3; K₂O: 13,5; ZnO: 10,5; P₂O₅: 3,5; CaO: 6,0;
F: 0,5; MgO: 1,3; ZrO₂: 2,0; CeO₂ : 0,6.

Zur Herstellung dieser Glaskeramik wurde ein Ausgangsglas entsprechender Zusammensetzung erschmolzen, gefrittet und zu einem Pulver aufgemahlen. Dieses Pulver wurde dann 4 Stunden lang bei 520°C und anschließend 1 Stunde lang bei 800°C wärmebehandelt. Zum Erhalt eines Beschichtungsmaterials, bei dem Sintertemperatur und Ausdehnungskoeffizient geeignet eingestellt sind, wurden 50 Gew.-% der Apatitglaskeramik mit 50 Gew.-% des erfindungsgemäßen Glases in Form von Pulvern mit einer mittleren Teilchengröße von weniger als 90 µm gemischt. Der thermische Ausdehnungskoeffizient dieses Dentalmaterials betrug 10,0 × 10⁻⁶K⁻¹. Die Sintertemperatur lag bei 730°C und wurde bei der Beschichtung des Gerüstes jeweils 1 Minute lang gehalten. Insgesamt wurden 5 Brände mit Materialauftrag bei 730°C bis zur Fertigstellung der Frontzahnbrücke durchgeführt. Der abschließende Glanzbrand wurde bei 720°C ohne Vakuum durchgeführt, um einen oberflächlichen Eigenglanz zu erzielen. Die erhaltene dreigliedrige Frontzahnbrücke zeigte einen homogenen Verbund zwischen Lithiumdisilikatgerüst und Sintermaterial. Es bildeten sich aufgrund des abgestimmten Wärmeausdehnungskoeffizienten, der niedrigen Sintertemperatur und der chemischen Verträglichkeit zwischen den einzelnen Komponenten keine Risse oder Sprünge in der Brücke.

## Patentansprüche

1. Tiefsinterndes Kalium-Zink-Silicat-Glas, **dadurch gekennzeichnet, daß** es die folgenden Komponenten enthält:
| Komponente | Gew.-% | | |
|---|---|---|---|
| SiO₂ | 60,0 | bis | 72,0 |
| Li₂O | 1,0 | bis | 5,0 |
| K₂O | 10,0 | bis | 23,0 |
| ZnO | 8,5 | bis | 20,0. |

2. Glas nach Anspruch 1, **dadurch gekennzeichnet, daß** es zusätzlich mindestens eine der folgenden Komponenten enthält:
| Komponente | Gew.-% | | |
|---|---|---|---|
| Na₂O | 0 | bis | 4,0 |
| MgO | 0 | bis | 4,0 |
| CaO | 0 | bis | 3,6 |
| SrO | 0 | bis | 3,0 |
| Al₂O₃ | 0 | bis | 8,0 |
| B₂O₃ | 0 | bis | 3,3 |
| La₂O₃ | 0 | bis | 3,0 |
| ZrO₂ | 0 | bis | 6,0 |
| TiO₂ | 0 | bis | 2,5 |
| CeO₂ | 0 | bis | 2,0 |
| SnO₂ | 0 | bis | 5,0 |
| P₂O₅ | 0 | bis | 1,0 |
| Tb₄O₇ | 0 | bis | 1, 8 |
| F | 0 | bis | 1,1 |

3. Glas nach Anspruch 1 oder 2, **dadurch gekennzeichnet, daß** die Mengen einiger Komponenten unabhängig voneinander wie folgt sind:
| Komponente | Gew.-% | | |
|---|---|---|---|
| SiO₂ | 62,0 | bis | 70,0 |
| Li₂O | 2,0 | bis | 5,0 |
| K₂O | 10,0 | bis | 20,0 |
| ZnO | 10,0 | bis | 19,0 |
| Na₂O | 0 | bis | 3,0 |
| MgO | 0 | bis | 3,0 |
| CaO | 0 | bis | 3,0 |
| SrO | 0 | bis | 3,0 |
| Al₂O₃ | 0 | bis | 6,0 |
| B₂O₃ | 0 | bis | 3,0 |
| La₂O₃ | 0 | bis | 2,0 |
| ZrO₂ | 0 | bis | 5,0 |
| TiO₂ | 0 | bis | 2,0 |
| CeO₂ | 0 | bis | 1,5 |
| SnO₂ | 0 | bis | 4,0 |
| P₂O₅ | 0 | bis | 0,8 |
| Tb₄O₇ | 0 | bis | 1,0 |
| F | 0 | bis | 1,0 |

4. Glas nach einem der Ansprüche 1 bis 3, **dadurch gekennzeichnet, daß** die Mengen einiger Komponenten unabhängig voneinander wie folgt sind:
| Komponente | Gew.-% | | |
|---|---|---|---|
| SiO₂ | 63,0 | bis | 69,0 |
| Li₂O | 3,0 | bis | 5,0 |
| K₂O | 11,0 | bis | 19,0 |
| ZnO | 10,0 | bis | 17,0 |
| Na₂O | 0 | bis | 2,5 |
| MgO | 0 | bis | 2,5 |
| CaO | 0 | bis | 2,5 |
| SrO | 0 | bis | 2,5 |
| Al₂O₃ | 0 | bis | 4,0 |
| B₂O₃ | 0 | bis | 2,0 |
| La₂O₃ | 0 | bis | 1,8 |
| ZrO₂ | 0 | bis | 4,0 |
| TiO₂ | 0 | bis | 1, 8 |
| CeO₂ | 0,1 | bis | 1,5 |
| SnO₂ | 0 | bis | 3,5 |
| P₂O₅ | 0 | bis | 0,5 |
| Tb₄O₇ | 0 | bis | 0,8 |
| F | 0 | bis | 0,8 |

5. Glas nach einem der Ansprüche 1 bis 4, **dadurch gekennzeichnet, daß** es bei thermischer Behandlung im Bereich von 600 °C bis 800°C für 1 Minute bis 1 Stunde nicht kristallisiert.

6. Glas nach einem der Ansprüche 1 bis 5, **dadurch gekennzeichnet, daß** es eine Sintertemperatur von weniger als 800°C und insbesondere von 760°C oder weniger aufweist.

7. Glas nach einem der Ansprüche 1 bis 6, **dadurch gekennzeichnet, daß** es einen linearen thermischen Ausdehnungskoeffizienten von weniger als 12,3 x 10⁻⁶ K⁻¹, gemessen im Bereich von 100 °C bis 400 °C, hat.

8. Dentalmaterial, **dadurch gekennzeichnet, daß** es das Glas gemäß einem der Ansprüche 1 bis 7 enthält.

9. Dentalmaterial nach Anspruch 8, **dadurch gekennzeichnet, daß** es zusätzlich eine Apatit-Glaskeramik enthält.

10. Dentalmaterial nach Anspruch 9 oder 10, **dadurch gekennzeichnet, daß** die Apatit-Glaskeramik die folgenden Komponenten enthält und die Hauptkristallphase durch Apatit-Kristalle gebildet ist:
| Komponente | Gew.-% | | |
|---|---|---|---|
| SiO₂ | 56,0 | bis | 65,0 |
| Li₂O | 1,8 | bis | 5,3 |
| K₂O | 9,0 | bis | 17,5 |
| ZnO | 9,0 | bis | 16,0 |
| CaO | 3,5 | bis | 10,5 |
| P₂O₅ | 2,0 | bis | 6,0 |
| F | 0,5 | bis | 1,0 |

11. Dentalmaterial nach einem der Ansprüche 8 bis 10, **dadurch gekennzeichnet, daß** es eine Sintertemperatur von weniger als 800°C und insbesondere von 760°C oder weniger aufweist.

12. Verwendung des Dentalmaterials gemäß einem der Ansprüche 8 bis 12 zur Beschichtung eines Substrates und insbesondere einer dentalen Restauration.

13. Verwendung nach Anspruch 13, wobei man das Dentalmaterial auf das Substrat aufbringt, ggf. in gewünschter Weise formt, und danach sintert, um eine auf dem Substrat fest haftende Beschichtung zu ergeben.

14. Verwendung nach Anspruch 12 oder 13, wobei ein Substrat auf Basis von keramischem oder glaskeramischem Werkstoff eingesetzt wird.

15. Verwendung nach Anspruch 14, wobei der glaskeramische Werkstoff eine Lithiumdisilicat-Glaskeramik ist, die die folgenden Komponenten enthält:
| Komponente | Gew.-% | | |
|---|---|---|---|
| SiO₂ | 57,0 | bis | 80,0 |
| Al₂O₃ | 0 | bis | 5,0 |
| La₂O₃ | 0, 1 | bis | 6,0 |
| MgO | 0 | bis | 5,0 |
| ZnO | 0 | bis | 8,0 |
| Li₂O | 11,0 | bis | 19,0 |
| P₂O₅ | 0 | bis | 11,0 |
wobei
(a) Al₂O₃ + La₂O₃ 0,1 bis 7,0 Gew.-% und
(b) MgO + ZnO 0,1 bis 9,0 Gew.-%
ausmachen.

16. Verwendung nach einem der Ansprüche 13 bis 15, wobei das Dentalmaterial auf das Substrat aufgebracht und bei Temperaturen von weniger als 800°C gesintert wird.

17. Geformtes Dentalprodukt, **dadurch gekennzeichnet, daß** es das Kalium-Zink-Silicat-Glas gemäß einem der Ansprüche 1 bis 7 oder das Dentalmaterial gemäß einem der Ansprüche 8 bis 11 enthält.

18. Geformtes Dentalprodukt nach Anspruch 17, **dadurch gekennzeichnet, daß** es eine dentale Restauration ist.

19. Geformtes Dentalprodukt nach Anspruch 17 oder 18, **dadurch gekennzeichnet, daß** es einen Kern auf Basis von keramischem oder glaskeramischem Werkstoff und eine darauf aufgebrachte Beschichtung mit Gehalt an dem Kalium-Zink-Silicat-Glas aufweist.

20. Geformtes Dentalprodukt nach Anspruch 19, **dadurch gekennzeichnet, daß** der glaskeramische Werkstoff eine Lithiumdisilicat-Glaskeramik ist.

## Claims

1. Low-temperature-sintering potassium-zinc-silicate glass, comprising the following components:
| Component | wt.% |
|---|---|
| SiO₂ | 60.0 to 72.0 |
| Li₂O | 1.0 to 5.0 |
| K₂O | 10.0 to 23.0 |
| ZnO | 8.5 to 20.0 |

2. Glass according to claim 1, additionally comprising at least one of the following components:
| Component | wt.% |
|---|---|
| Na₂O | 0 to 4.0 |
| MgO | 0 to 4.0 |
| CaO | 0 to 3.6 |
| SrO | 0 to 3.0 |
| Al₂O₃ | 0 to 8.0 |
| B₂O₃ | 0 to 3.3 |
| La₂O₃ | 0 to 3.0 |
| ZrO₂ | 0 to 6.0 |
| TiO₂ | 0 to 2.5 |
| CeO₂ | 0 to 2.0 |
| SnO₂ | 0 to 5.0 |
| P₂O₅ | 0 to 1.0 |
| Tb₄O₇ | 0 to 1.8 |
| F | 0 to 1.1 |

3. Glass according to claim 1 or 2, wherein the amounts of some components, independently of each other, are as follows:
| Component | wt.% |
|---|---|
| SiO₂ | 62.0 to 70.0 |
| Li₂O | 2.0 to 5.0 |
| K₂O | 10.0 to 20.0 |
| ZnO | 10.0 to 19.0 |
| Na₂O | 0 to 3.0 |
| MgO | 0 to 3.0 |
| CaO | 0 to 3.0 |
| SrO | 0 to 3.0 |
| Al₂O₃ | 0 to 6.0 |
| B₂O₃ | 0 to 3.0 |
| La₂O₃ | 0 to 2.0 |
| ZrO₂ | 0 to 5.0 |
| TiO₂ | 0 to 2.0 |
| CeO₂ | 0 to 1.5 |
| SnO₂ | 0 to 4.0 |
| P₂O₅ | 0 to 0.8 |
| Tb₄O₇ | 0 to 1.0 |
| F | 0 to 1.0 |

4. Glass according to one of claims 1 to 3, wherein the amounts of some components, independently of each other, are as follows:
| Component | wt.% |
|---|---|
| SiO₂ | 63.0 to 69.0 |
| Li₂O | 3.0 to 5.0 |
| K₂O | 11.0 to 19.0 |
| ZnO | 10.0 to 17.0 |
| Na₂O | 0 to 2.5 |
| MgO | 0 to 2.5 |
| CaO | 0 to 2.5 |
| SrO | 0 to 2.5 |
| Al₂O₃ | 0 to 4.0 |
| B₂O₃ | 0 to 2.0 |
| La₂O₃ | 0 to 1.8 |
| ZrO₂ | 0 to 4.0 |
| TiO₂ | 0 to 1.8 |
| CeO₂ | 0.1 to 1.5 |
| SnO₂ | 0 to 3.5 |
| P₂O₅ | 0 to 0.5 |
| Tb₄O₇ | 0 to 0.8 |
| F | 0 to 0.8 |

5. Glass according to one of claims 1 to 4, wherein said glass does not crystallise during thermal treatment in the range of 600 °C to 800 °C for 1 minute to 1 hour.

6. Glass according to one of claims 1 to 5, wherein said glass has a sintering temperature of less than 800 °C and in particular of 760 °C or less.

7. Glass according to one of claims 1 to 6, wherein said glass has a linear thermal coefficient of expansion of less than 12.3*10⁻⁶K⁻¹, measured in the range of 100 °C to 400 °C.

8. Dental material, comprising the glass according to any one of claims 1 to 7.

9. Dental material according to claim 8, additionally comprising an apatite glass ceramic.

10. Dental material according to claim 9 or 10, wherein the apatite glass ceramic comprises the following components and the main crystal phase is formed by apatite crystals:
| Component | wt.% |
|---|---|
| SiO₂ | 56.0 to 65.0 |
| Li₂O | 1.8 to 5.3 |
| K₂O | 9.0 to 17.5 |
| ZnO | 9.0 to 16.0 |
| CaO | 3.5 to 10.5 |
| P₂O₅ | 2.0 to 6.0 |
| F | 0.5 to 1.0 |

11. Dental material according to one of claims 8 to 10, wherein said material has a sintering temperature of less than 800 °C and in particular of 760 °C or less.

12. Use of the dental material according to one of claims 8 to 12 for coating a substrate and in particular a dental restoration.

13. Use according to claim 13, wherein the dental material is applied to the substrate, optionally shaped in the desired manner, and then sintered, in order to produce a coating adhering firmly to the substrate.

14. Use according to claim 12 or 13, wherein a substrate based on ceramic or glass ceramic material is used.

15. Use according to claim 14, wherein the glass ceramic material is a lithium disilicate glass ceramic comprising the following components:
| Component | wt.% |
|---|---|
| SiO₂ | 57.0 to 80.0 |
| Al₂O₃ | 0 to 5.0 |
| La₂O₃ | 0.1 to 6.0 |
| MgO | 0 to 5.0 |
| ZnO | 0 to 8.0 |
| Li₂O | 11.0 to 19.0 |
| P₂O₅ | 0 to 11.0 |
wherein
(a) Al₂O₃ + La₂O₃ account for 0.1 to 7.0 wt.% and
(b) MgO + ZnO account for 0.1 to 9.0 wt.%.<Component<wt.-%

16. Use according to one of claims 13 to 15, wherein the dental material is applied to the substrate and sintered at temperatures of less than 800 °C.

17. Shaped dental product comprising the potassium-zinc-silicate glass according to one of claims 1 to 7 or the dental material according to one of claims 8 to 11.

18. Shaped dental product according to claim 17, which is a dental restoration.

19. Shaped dental product according to claim 17 or 18, exhibiting a core based on ceramic or glass ceramic material and a coating applied to it comprising the potassium-zinc-silicate glass.

20. Shaped dental product according to claim 19, wherein the glass ceramic material is a lithium disilicate glass ceramic.

## Revendications

1. Verre de silicate de potassium-zinc pouvant fritter à basse température, **caractérisé en ce qu'**il contient les composants suivants :
| Composant | | % en poids | |
|---|---|---|---|
| SiO₂ | 60,0 | à | 72,0 |
| Li₂O | 1,0 | à | 5,0 |
| K₂O | 10,0 | à | 23,0 |
| ZnO | 8,5 | à | 20,0 |

2. Verre selon la revendication 1, **caractérisé en ce qu'**il contient, en plus, au moins l'un des composants suivants :
| Composant | | % en poids | |
|---|---|---|---|
| Na₂O | 0 | à | 4,0 |
| MgO | 0 | à | 4,0 |
| CaO | 0 | à | 3,6 |
| SrO | 0 | à | 3,0 |
| Al₂O₃ | 0 | à | 8,0 |
| B₂O₃ | 0 | à | 3,3 |
| La₂O₃ | 0 | à | 3,0 |
| ZrO₂ | 0 | à | 6,0 |
| TiO₂ | 0 | à | 2,5 |
| CeO₂ | 0 | à | 2,0 |
| SnO₂ | 0 | à | 5,0 |
| P₂O₅ | 0 | à | 1,0 |
| Tb₄O₇ | 0 | à | 1,8 |
| F | 0 | à | 1,1 |

3. Verre selon la revendication 1 ou 2, **caractérisé en ce que** les quantités de quelques-uns des composants sont, indépendamment l'un de l'autre, les suivantes :
| Composant | | %) en poids | |
|---|---|---|---|
| SiO₂ | 62,0 | à | 70,0 |
| Li₂O | 2,0 | à | 5,0 |
| K₂O | 10,0 | à | 20,0 |
| ZnO | 10,0 | à | 19,0 |
| Na₂O | 0 | à | 3,0 |
| MgO | 0 | à | 3,0 |
| CaO | 0 | à | 3,0 |
| SrO | 0 | à | 3,0 |
| Al₂O₃ | 0 | à | 6,0 |
| B₂O₃ | 0 | à | 3,0 |
| La₂O₃ | 0 | à | 2,0 |
| ZrO₂ | 0 | à | 5,0 |
| TiO₂ | 0 | à | 2,0 |
| CeO₂ | 0 | à | 1,5 |
| SnO₂ | 0 | à | 4,0 |
| P₂O₅ | 0 | à | 0,8 |
| Tb₄O₇ | 0 | à | 1,0 |
| F | 0 | à | 1,0 |

4. Verre selon l'une quelconque des revendications 1 à 3, **caractérisé en ce que** les quantités de quelques-uns des composants sont, indépendamment l'un de l'autre, les suivantes :
| Composant | | % en poids | |
|---|---|---|---|
| SiO₂ | 63,0 | à | 69,0 |
| Li₂O | 3,0 | à | 5,0 |
| K₂O | 11,0 | à | 19,0 |
| ZnO | 10,0 | à | 17,0 |
| Na₂O | 0 | à | 2,5 |
| MgO | 0 | à | 2,5 |
| CaO | 0 | à | 2,5 |
| SrO | 0 | à | 2,5 |
| Al₂O₃ | 0 | à | 4,0 |
| B₂O₃ | 0 | à | 2,0 |
| La₂O₃ | 0 | à | 1,8 |
| ZrO₂ | 0 | à | 4,0 |
| TiO₂ | 0 | à | 1,8 |
| CeO₂ | 0,1 | à | 1,5 |
| SnO₂ | 0 | à | 3,5 |
| P₂O₅ | 0 | à | 0,5 |
| Tb₄O₇ | 0 | à | 0,8 |
| F | 0 | à | 0,8 |

5. Verre selon l'une quelconque des revendications 1 à 4, **caractérisé en ce qu'**il ne cristallise pas lors d'un traitement thermique effectué dans la plage de 600 °C à 800 °C sur une période de 1 minute à 1 heure.

6. Verre selon l'une quelconque des revendications 1 à 5, **caractérisé en ce qu'**il présente une température de frittage inférieure à 800 °C et, en particulier, égale ou inférieure à 760 °C.

7. Verre selon l'une quelconque des revendications 1 à 6, **caractérisé en ce qu'**il présente un coefficient de dilatation thermique linéaire inférieur à 12,3 x 10⁻⁶K⁻¹, mesuré dans la plage de 100 °C à 400 °C.

8. Matériau dentaire, **caractérisé en ce qu'**il contient le verre selon l'une quelconque des revendications 1 à 7.

9. Matériau dentaire selon la revendication 8, **caractérisé en ce qu'**il contient, en outre, une vitrocéramique à base d'apatite.

10. Matériau dentaire selon la revendication 9 ou 10, **caractérisé en ce que** la vitrocéramique à base d'apatite contient les composants suivants et la phase cristalline principale est formée de cristaux d'apatite :
| Composant | | % en poids | |
|---|---|---|---|
| SiO₂ | 56,0 | à | 65,0 |
| Li₂O | 1,8 | à | 5,3 |
| K₂O | 9,0 | à | 17,5 |
| ZnO | 9,0 | à | 16,0 |
| CaO | 3,5 | à | 10,5 |
| P₂O₅ | 2,0 | à | 6,0 |
| F | 0,5 | à | 1,0 |

11. Matériau dentaire selon l'une quelconque des revendications 8 à 10, **caractérisé en ce qu'**il présente une température de frittage inférieure à 800 °C et, en particulier, égale ou inférieure à 760 °C.

12. Utilisation du matériau dentaire selon l'une quelconque des revendications 8 à 12, pour revêtir un substrat et, en particulier, une restauration dentaire.

13. Utilisation selon la revendication 13, dans laquelle on applique le matériau dentaire sur le substrat, éventuellement, on le façonne de la manière souhaitée, puis on le soumet à un frittage dans le but d'obtenir un revêtement adhérant solidement au substrat.

14. Utilisation selon la revendication 12 ou 13, dans laquelle on utilise un substrat à base de matériau céramique ou vitrocéramique.

15. Utilisation selon la revendication 14, dans laquelle le matériau vitrocéramique est une vitrocéramique de disilicate de lithium qui contient les composants suivants :
| Composant | | % en poids | |
|---|---|---|---|
| SiO₂ | 57,0 | à | 80,0 |
| Al₂O₃ | 0 | à | 5,0 |
| La₂O₃ | 0,1 | à | 6,0 |
| MgO | 0 | à | 5,0 |
| ZnO | 0 | à | 8,0 |
| Li₂O | 11,0 | à | 19,0 |
| P₂O₅ | 0 | à | 11,0 |
où
(a) Al₂O₃ + La₂O₃ totalisent 0,1 à 7,0 % en poids et
(b) MgO + ZnO totalisent 0,1 à 9,0 % en poids

16. Utilisation selon l'une quelconque des revendications 13 à 15, dans laquelle le matériau dentaire est appliqué sur le substrat et soumis à un frittage à des températures inférieures à 800 °C.

17. Produit dentaire façonné, **caractérisé en ce qu'**il contient le verre de silicate de potassium-zinc selon l'une quelconque des revendications 1 à 7, ou le matériau dentaire selon l'une quelconque des revendications 8 à 11.

18. Produit dentaire façonné selon la revendication 17, **caractérisé en ce qu'**il s'agit d'une restauration dentaire.

19. Produit dentaire façonné selon la revendication 17 ou 18, **caractérisé en ce qu'**il présente un noyau à base de matériau céramique ou vitrocéramique et un revêtement appliqué sur celui-ci et présentant une certaine teneur en verre de silicate de potassium-zinc.

20. Produit dentaire façonné selon la revendication 19, **caractérisé en ce que** le matériau vitrocéramique est une vitrocéramique à base de disilicate de lithium.
